# EUROPÄISCHE PATENTSCHRIFT

(11) **EP 1 339 791 B1**
(45) Veröffentlichungstag und Bekanntmachung des Hinweises auf die Patenterteilung: **25.02.2015**
(21) Anmeldenummer: 01998591.0
(22) Anmeldetag: 28.11.2001
(51) Int. Cl.: C08L 51/00, C08F 291/00, C08F 265/04, C08F 271/02

(54) **GEPFROPFTE COPOLYMERE AUF BASIS VON ACRYOYLDIMETHYLTAURINSÄURE**
ACRYOYLDIMETHYLTAURINE ACID-BASED GRAFTED COPOLYMERS
COPOLYMERE GREFFE A BASE D'ACIDE ACRYLOYLDIMETHYLETAURINE

(30) Priorität: 01.12.2000 DE 10059832
(43) Veröffentlichungstag der Anmeldung: 03.09.2003
(73) Patentinhaber: Clariant Produkte (Deutschland) GmbH, 65929 Frankfurt am Main (DE)
(72) Erfinder: MORSCHHÄUSER, Roman, 55122 Mainz (DE); LÖFFLER, Matthias, 65527 Niedernhausen (DE)
(86) Internationale Anmeldenummer: PCT/EP2001/013857
(87) Internationale Veröffentlichungsnummer: WO 2002/044269

(56) Entgegenhaltungen:
- EP-A- 0 356 241
- EP-A- 0 815 828
- EP-A- 0 815 844
- EP-A- 0 815 845
- EP-A- 0 816 403
- US-A- 3 931 089
- US-A- 4 521 578
- US-A- 5 368 850

## Beschreibung

Die vorliegende Erfindung betrifft Copolymere auf Basis von Acryloyldimethyltaurinsäure bzw. Acryloyldimethyltauraten.

In den letzten Jahren erlangten wasserlösliche Polymere eine immer größer werdende Bedeutung in Industrie und Wissenschaft. Polyelektrolyte nehmen dabei mengenmäßig einen sehr großen Teil der jährlichen Gesamtproduktion ein. Sie finden z.B. Anwendung in der Papierverarbeitung, der Waschmittelindustrie, der Textilverarbeitung, der Erdölgewinnung oder als wichtige Kosmetikrohstoffe.

Im kosmetischen Bereich kommt Polyelektrolyten eine tragende Rolle zu. Neben wasserlöslichen, oberflächenaktiven Stoffen gibt es in diesem Bereich einen hohen Bedarf an wasser- und ölverdickenden Systemen. Derartige Verdicker, insbesondere die auf Basis der Polyacrylsäure hergestellten "Superabsorber", sind seit ihrer Entwicklung in den 70iger Jahren aus dem Hygienebereich nicht mehr wegzudenken. In ihren vernetzten Varianten werden teil- oder vollneutralisierte Polyacrylsäuren und deren wasserlösliche Copolymere in vielen Kosmetikformulierungen als Konsistenzgeber eingesetzt. Die Vielfalt der möglichen Strukturen und die damit verbundenen vielfältigen Anwendungsmöglichkeiten drücken sich nicht zuletzt in einer Vielzahl von Patenten aus, die seit Mitte der 70iger Jahre weltweit angemeldet wurden.

In den 90iger Jahren wurden neuartige Verdicker auf Basis von Acryloyldimethyltaurinsäure (AMPS) bzw. deren Salzen in den Markt eingeführt (EP 816 403 EP1069142, DE19907587, EP0815845, EP1059316 und WO 98/ 00094). Sowohl als Homo- als auch in Form der Copolymere (^{®}Aristoflex AVC, Clariant GmbH) sind derartige Verdicker den entsprechenden Polycarboxylaten (Carbopole) in vieler Hinsicht überlegen. Beispielsweise zeigen Verdickersysteme auf Basis von AMPS hervorragende Eigenschaften in pH-Bereichen unterhalb von pH 6, also in einem pH-Bereich, in dem mit herkömmlichen Polycarboxylat-Verdickern nicht mehr gearbeitet werden kann. Zudem führt die Mikrogelstruktur solcher Verdicker zu einem besonders angenehmen Hautgefühl. Die leichte Verarbeitbarkeit und das günstige toxikologische Profil des Hauptmonomeren verleihen diesen Verdickern ein hohes Anwendungspotential.

Nachteilig an Verdickern auf Basis von Acryloyldimethyltaurinsäure ist die häufig auftretende Opaleszenz ihrer verdünnten, wässrigen Gele. Ursächlich für die Opaleszenz ist die starke Streuung des sichtbaren Lichtes an übervernetzten Polymeranteilen, die während der Polymerisation entstehen und in Wasser nur unzureichend gequollen vorliegen.

Überraschend wurde nun gefunden, dass Copolymere auf Basis von Acryloyldimethyltaurinsäure (AMPS), die dadurch erhältlich sind, dass die Polymerisation in Gegenwart eines polymeren Additivs (c) erfolgt, sehr gute Verdickereigenschaften und eine klare Optik zeigen.

Gegenstand der Erfindung sind wasserlösliche oder wasserquellbare Copolymere, erhältlich durch radikalische Copolymerisation von
A) Acryloyldimethyltaurinsäure und/oder Acryloyldimethyltauraten und
B) gegebenenfalls einem oder mehreren weiteren olefinisch ungesättigten, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen, wobei die Copolymerisation
C) in Gegenwart mindestens eines polymeren Additivs mit einem zahlenmittleren Molekulargewicht von 200 g/mol bis 10⁹ g/mol, ausgewählt aus Homo- oder Copolymeren aus N-Vinylformamid, N-Vinylacetamid, N-Vinylpyrrolidon, Acryloyldimethyl-taurinsäure, N-Vinylcaprolactam, N-Vinylmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimethylammoniumchlorid (MAPTAC);
erfolgt,
dadurch gekennzeichnet, dass die Copolymere durch Fällungspolymerisation in tert.-Butanol hergestellt werden.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure handeln. Bevorzugt werden die Li⁺-, Na⁺-, K⁺-, Mg⁺⁺-, Ca⁺⁺-, Al⁺⁺⁺- und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80 %.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.

Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 22.

Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.

Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁ - C₂₂)-Alkylreste handeln kann, die gegebenenfalls mit bis zu 3 (C₂ - C₁₀)-Hydroxyalkylgruppen besetzt sein können. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100 % betragen.

Als Comonomere weiterhin bevorzugt sind offenkettige N-Vinylamide, bevorzugt N-Vinylformamid (VIFA), N-Vinyl-methylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid; Hydroxyethylmethacrylamid, Hydroxypropylmethacrylamid und Bernsteinsäuremono-[2-(methacryloyloxy)-ethylester]; N, N-Dimethylaminomethacrylat; Diethylamino-methylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.

Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%.

Wesentlich für die Erfindung ist, dass die Copolymerisation in Gegenwart mindestens eines polymeren Additivs C) durchgeführt wird, wobei das Additiv C) vor der eigentlichen Copolymerisation dem Polymerisationsmedium ganz- oder teilweise gelöst zugegeben wird. Die Verwendung von mehreren Additiven C) ist ebenfalls erfindungsgemäß. Vernetzte Additive C) können ebenfalls verwendet werden.

Die Additive C) bzw. deren Mischungen müssen lediglich ganz oder teilweise im gewählten Polymerisationsmedium löslich sein.

Während des eigentlichen Polymerisationsschrittes hat das Additiv C) mehrere Funktionen. Einerseits verhindert es im eigentlichen Polymerisationsschritt die Bildung übervernetzter Polymeranteile im sich bildenden Copolymerisat und andererseits wird das Additiv C) gemäß dem allgemein bekannten Mechanismus der Pfropfcopolymerisation statistisch von aktiven Radikalen angegriffen. Dies führt dazu, dass je nach Additiv C) mehr oder weniger große Anteile davon in die Copolymere eingebaut werden. Zudem besitzen geeignete Additive C) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive C) hergestellt wurden, zeigen solche, die unter Zusatz von Additiven C) hergestellt wurden, vorteilhafterweise eine signifikant höhere Viskosität in wässriger Lösung.

Bevorzugt als Additive C) sind in Wasser und/oder Alkoholen lösliche Homo- und Copolymere. Unter Copolymeren sind dabei auch solche mit mehr als zwei verschiedenen Monomertypen zu verstehen.

Additive C) sind Homo- und Copolymere aus N-Vinylformamid, N-Vinylacetamid, N-Vinylpyrrolidon, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC) .

Insbesondere bevorzugt als Additive C) sind Polyvinylpyrrolidone (z.B. K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.

Das Molekulargewicht der Additive C) beträgt bevorzugt 10² bis 10⁷ g/mol, besonders bevorzugt 0,5*10⁴ bis 10⁶ g/mol.

Die Einsatzmenge des polymeren Additivs C) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.

Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.

Besonders bevorzugt als Vernetzer ist Trimethylolpropantriacrylat (TMPTA).

Der Gewichtsanteil an vernetzenden Comonomeren, bezogen auf die Gesamtmasse der Copolymere, beträgt bevorzugt bis 20 Gew.-%, besonders bevorzugt 0,05 bis
10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen PolymerisationsInitiatoren, wie organische Peroxide, z.B. Benzoylperoxid, tert.-Butylhydroperoxid, Methylethylketonperoxid, Cumolhydroperoxid, Dilauroylperoxid (DLP) oder Azoinitiatoren, wie z.B. Azodiisobutyronitril (AIBN), verwendet werden.

Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Die Polymerisationsreaktion kann z.B. als Fällungspolymerisation, Emulsionspolymerisation, Substanzpolymerisation, Lösungspolymerisation oder Gelpolymerisation geführt werden. Besonders vorteilhaft für das Eigenschaftsprofil der erfindungsgemäßen Copolymere ist die Fällungspolymerisation, bevorzugt in tert.-Butanol.

Die erfindungsgemäßen multifunktionalen Polymere besitzen eine große Strukturvielfalt und folglich breite potentielle Einsatzmöglichkeiten, die auf nahezu jede Fragestellung, bei der Grenzflächen- bzw. Oberflächeneffekte eine Rolle spielen, zugeschnitten werden können. Insbesondere soll auch auf die Anwendungsmöglichkeiten im Bereich der Kosmetik, z.B. als Verdicker, hingewiesen werden. Der Begriff "maßgeschneiderte Polymere" beschreibt bildlich die Möglichkeiten, die diese neue Polymerklasse dem Anwender an die Hand gibt.

Nachfolgende Beispiele sollen die Erfindung näher erläutern, ohne sie jedoch darauf einzuschränken.

### Beispiel 1

| Reaktanden | Menge (g) |
|---|---|
| NH₃-neutralisiertes AMPS | 80 |
| t-Butanol | 300 |
| TMPTA | 1,8 |
| Dilauroylperoxid (Initiator) | 1 |
| Poly-N-Vinylpyrrolidon (^{®}K-15, BASF) | 5 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von DLP initiiert. Das Polymer wurde durch Absaugen des Lösungsmittels und durch anschließende Vakuumtrocknung isoliert.

### Beispiel 2

| Reaktanden | Menge (g) |
|---|---|
| NH₃-neutralisiertes AMPS | 80 |
| N-Vinylpyrrolidon | 5 |
| Wasser | 300 |
| AMA | 0,9 |
| Na₂S₂Oₐ (Initiator) | 1 |
| Poly-N-Vinylpyrrolidon (^{®}K-30, BASF) | 10 |

Das Polymer wurde nach dem Gelpolymerisationsverfahren in Wasser hergestellt. Dabei wurden die Monomere in Wasser gelöst, die Reaktionsmischung inertisiert und anschließend die Reaktion nach Anheizen durch Zugabe von Natriumperoxodisulfat initiiert. Das Polymergel wurde anschließend zerkleinert und das Polymer durch Vakuumtrocknung isoliert.

### Beispiel 3

| Reaktanden | Menge (g) |
|---|---|
| AMPS | 80 |
| Vinylacetat | 20 |
| Cyclohexan | 200 |
| Wasser | 300 |
| ^{®}Span 80 | 1 |
| Na₂S₂O₈ (Initiator) | 1 |
| Poly[N-vinylpyrrolidon-co-acrylsäure] (30/70) | 4 |

Das Polymer wurde nach dem Emulsionsverfahren in Wasser hergestellt. Dabei wurden die Monomere in Wasser/Cyclohexan unter Verwendung von ^{®}Span 80 emulgiert, die Reaktionsmischung mittels N₂ inertisiert und anschließend die Reaktion nach Anheizen durch Zugabe von Natriumperoxodisulfat gestartet. Die Polymeremulsion wurde anschließend eingedampft und dadurch das Polymer isoliert.

### Beispiel 4

| Reaktanden | Menge (g) |
|---|---|
| NH₃-neutralisiertes AMPS | 80 |
| t-Butanol | 300 |
| N-Vinylformamid | 20 |
| TMPTA | 1,8 |
| ABAH (Initiator) | 1 |
| Poly[N-vinylcaprolacton-co-acrylsäure] (10/90) | 10 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von ABAH initiiert. Das Polymer wurde durch Absaugen des Lösungsmittels und durch anschließende Vakuumtrocknung isoliert.

### Beispiel 5

| Reaktand | Menge (g) |
|---|---|
| Na-neutralisiertes AMPS | 80 |
| Wasser | 300 |
| TMPTA | 1,8 |
| H₂O₂/Eisen (Initiator) | 1 |
| Poly[N-Vinylfomamid] | 8 |

Das Polymer wurde nach dem Lösungsverfahren in Wasser hergestellt. Dabei wurden die Monomere in Wasser gelöst, die Reaktionsmischung inertisiert und anschließend die Reaktion nach Anheizen durch ein geeignetes Redoxpaar initiiert. Die Polymerlösung wurde anschließend eingedampft und dadurch das Polymer isoliert.

### Chemische Bezeichnung der eingesetzten Produkte

- TMPTA: Trimethylolpropantriacrylat
- AMA: Allylmethacrylat
- ABAH: Azobisamidopropylhydrochlorid
- AIBN: Azoisobutyronitril

## Patentansprüche

1. Wasserlösliche oder wasserquellbare Copolymere, erhältlich durch radikalische Copolymerisation von
A) Acryloyldimethyltaurinsäure und/oder Acryloyldimethyltauraten und
B) gegebenenfalls einem oder mehreren weiteren olefinisch ungesättigten, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen, wobei die Copolymerisation
C) in Gegenwart mindestens eines polymeren Additivs mit einem zahlenmittleren Molekulargewicht von 200 g/mol bis 10⁹ g/mol, ausgewählt aus Homo- oder Copolymeren aus N-Vinylformamid, N-Vinylacetamid, N-Vinylpyrrolidon, Acryloyldimethyl-taurinsäure, N-Vinylcaprolactam, N-Vinylmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimethylammoniumchlorid (MAPTAC); erfolgt,
**dadurch gekennzeichnet, dass** die Copolymere durch Fällungspolymerisation in tert.-Butanol hergestellt werden.

2. Copolymere nach Anspruch 1, **dadurch gekennzeichnet, dass** sie zusätzlich ein oder mehrere Comonomere B) enthalten.

3. Copolymere nach Anspruch 2, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

4. Copolymere nach mindestens einem der Ansprüche 1 bis 3, **dadurch gekennzeichnet, dass** es sich bei den polymeren Additiven C) um Poly(N-Vinylformamide), Poly(N-Vinylcaprolactame) und Copolymere aus N-Vinylpyrrolidon, N-Vinylformamid und/oder Acrylsäure handelt.

5. Copolymere nach mindestens einem der Ansprüche 1 bis 4, **dadurch gekennzeichnet, dass** sie vernetzt sind.

## Claims

1. A water-soluble or water-swellable copolymer obtainable by free-radical copolymerization of
A) acryloyldimethyltaurine and/or acryloyldimethyltaurates, and
B) if desired, one or more other olefinically unsaturated, optionally crosslinking comonomers containing at least one oxygen, nitrogen, sulfur or phosphorus atom and possessing a molecular weight of less than 500 g/mol, the copolymerization
C) taking place in the presence of at least one polymeric additive having a number-average molecular weight of from 200 g/mol to 10⁹ g/mol, selected from homopolymers and copolymers of N-vinylformamide, N-vinylacetamide, N-vinylpyrrolidone, acryloyldimethyltaurine, N-vinylcaprolactam, N-vinylmethylacetamide, acrylamide, acrylic acid, methacrylic acid, N-vinylmorpholide, hydroxymethyl methacrylate, diallyldimethylammonium chloride (DADMAC) and/or [2-(methacryloyloxy)ethyl]trimethylammonium chloride (MAPTAC);
prepared by precipitation polymerization in tert-butanol.

2. The copolymer as claimed in claim 1, further containing one or more comonomers B).

3. The copolymer as claimed in claim 2, wherein the comonomers B) comprise unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate; styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

4. The copolymer as claimed in at least one of claims 1 to 3, wherein the polymeric additives C) are poly(N-vinylformamides), poly(N-vinylcaprolactams), and copolymers of N-vinylpyrrolidone, N-vinylformamide and/or acrylic acid.

5. The copolymer as claimed in at least one of claims 1 to 4, which is crosslinked.

## Revendications

1. Copolymères hydrosolubles ou aptes à gonfler dans l'eau, pouvant être obtenus par copolymérisation radicalaire
A) d'acide acryloyldiméthyltaurique et/ou d'acryloyldiméthyltaurates et
B) éventuellement d'un ou de plusieurs autres comonomères à insaturation oléfinique, éventuellement réticulables, qui comportent au moins un atome d'oxygène, d'azote, de soufre ou de phosphore et ont une masse moléculaire inférieure à 500 g/mole, la copolymérisation s'effectuant
C) en présence d'au moins un additif polymère ayant une masse moléculaire moyenne en nombre de 200 g/mole à 10⁹ g/mole, choisi parmi des homopolymères ou copolymères de N-vinylformamide, N-vinylacétamide, N-vinylpyrrolidone, acide acryloyldiméthyltaurique, N-vinylcaprolactame, N-vinylméthylacétamide, acrylamide, acide acrylique, acide méthacrylique, N-vinylmorpholide, méthacrylate d'hydroxyméthyle, chlorure de diallyldiméthylammonium (DADMAC) et/ou chlorure de [2-(méthacryloyloxy)éthyl]triméthylammonium (MAPTAC) ;
**caractérisés en ce que** les copolymères sont préparés par polymérisation par précipitation dans du tert-butanol.

2. Copolymères selon la revendication 1, **caractérisés en ce qu'**ils contiennent en plus un ou plusieurs comonomères B).

3. Copolymères selon la revendication 2, **caractérisés en ce que** les comonomères B) consistent en des acides carboxyliques insaturés, des sels d'acides carboxyliques insaturés, des anhydrides d'acides carboxyliques insaturés, des esters d'acides carboxyliques insaturés avec des alcools aliphatiques, oléfiniques, cycloaliphatiques, arylaliphatiques ou aromatiques ayant de 1 à 22 atomes de carbone, des N-vinylamides à chaîne ouverte, des N-vinylamides cycliques ayant une taille de cycle de 3 à 9, des amides de l'acide acrylique, des amides de l'acide méthacrylique, des amides d'acides acryliques substitués, des amides d'acides méthacryliques substitués, la 2-vinylpyridine, la 4-vinylpyridine, l'acétate de vinyle ; le styrène, l'acrylonitrile, le chlorure de vinyle, le chlorure de vinylidène, le tétrafluoroéthylène, l'acide vinylphosphonique ou ses esters ou sels, l'acide vinylsulfonique ou ses esters ou sels, l'acide allylphosphonique ou ses esters ou sels et/ou l'acide méthallylsulfonique ou ses esters ou sels.

4. Copolymères selon au moins l'une quelconque des revendications 1 à 3, **caractérisés en ce que** les additifs polymères C) consistent en des poly(N-vinylformamide)s, poly(N-vinylcaprolactame)s et copolymères de N-vinylpyrrolidone, N-vinylformamide et/ou acide acrylique.

5. Copolymères selon au moins l'une quelconque des revendications 1 à 4, **caractérisés en ce qu'**ils sont réticulés.
